# EUROPEAN PATENT SPECIFICATION

(11) **EP 1 702 620 B1**
(45) Date of publication and mention of the grant of the patent: **08.01.2014**
(21) Application number: 04799849.7
(22) Date of filing: 19.11.2004
(51) Int. Cl.: A61K 31/7115, A61K 48/00, C12N 5/10, A01K 67/027, A61P 37/04

(54) **IMMUNOPOTENTIATOR AND METHOD OF ENHANCING IMMUNOLOGICAL ACTIVITY WITH THE SAME**
IMMUNOPOTENTIATOR UND VERFAHREN ZUR VERBESSERUNG DER IMMUNOLOGISCHEN WIRKUNG DAMIT
IMMUNOPOTENTIALISATEUR ET SON EMPLOI DANS LE CADRE D'UNE METHODE D'ACCENTUATION DE L'ACTIVITE IMMUNOLOGIQUE

(30) Priority: 25.12.2003 JP 2003431007
(43) Date of publication of application: 20.09.2006
(73) Proprietor: Japan Science and Technology Agency, Kawaguchi-shi, Saitama 332-0012 (JP)
(72) Inventor: KAMIYA, Hiroyuki, Kita-ku Sapporoshi Hokkaido 00100 (JP); HARASHIMA, Hideyoshi, Sapporo-shi, Hokkaido 001-0907 (JP); TSUCHIYA, Hiroyuki, Sapporo-shi, Hokkaido 001-0013 (JP)
(74) Representative: Owen, Deborah Jane
(86) International application number: PCT/JP2004/017647
(87) International publication number: WO 2005/063264

(56) References cited:
- WO-A2-02/26757
- JP-A- 3 135 918
- JP-A- 9 323 979
- JP-A- 10 506 265
- JP-A- 2003 535 146
- BALLAS Z K ET AL: "INDUCTION OF NK ACTIVITY IN MURINE AND HUMAN CELLS BY CPG MOTIFS INOLIGODEOXYNUCLEOTIDES AND BACTERIAL DNA" JOURNAL OF IMMUNOLOGY, AMERICAN ASSOCIATION OF IMMUNOLOGISTS, US, vol. 157, no. 5, 1 September 1996 (1996-09-01), pages 1840-1845, XP002053416 ISSN: 0022-1767
- LIANG ET AL: "Activation of human B cells by phosphorothioate oligodeoxynucleotides" JOURNAL OF CLINICAL INVESTIGATION, AMERICAN SOCIETY FOR CLINICAL INVESTIGATION, US, vol. 98, no. 5, 1 September 1996 (1996-09-01), pages 1119-1129, XP002130608 ISSN: 0021-9738
- DATABASE BIOSIS [Online] BIOSCIENCES INFORMATION SERVICE, PHILADELPHIA, PA, US; November 2003 (2003-11), TREVANI ANALIA S ET AL: "Bacterial DNA activates human neutrophils by a CpG-independent pathway." XP002528938 Database accession no. PREV200300551133 & EUROPEAN JOURNAL OF IMMUNOLOGY, vol. 33, no. 11, November 2003 (2003-11), pages 3164-3174, ISSN: 0014-2980
- BRUNNER C. ET AL.: 'Enhanced dendritic cell maturation by TNF-alpha or cytidine-phosphate-guanosine DNA drives T cell activation in vitro and therapeutic anti-tumor immune responses in vivo' J. IMMUNOL. vol. 165, no. 11, 01 December 2000, pages 6278 - 6286, XP002983687

## Description

### Technical Field

The invention of this application relates to an immunopotentiator and a method for enhancing an immunoactivity using the same. More specifically, the invention of this application relates to an immunopotentiator using a nucleic acid containing a special nucleic acid base, a derivative thereof or a plasmid having the nucleic acid containing the special nucleic acid base, and a method for enhancing an immunoactivity using the same.

### Background Art

An immunoreaction in mammals such as mice, rats and rabbits is one of bioreactions important for preventing invasion of foreign matters, e.g., microbes such as bacteria and viruses, pollens and chemicals into the living body and infection thereof. In the immunoreaction, "adaptive immune" has an immunoreaction that mainly conducts an action of immune antibodies which are specifically bound to these foreign matters in a diversified manner to make them non-toxic. Further, in recent years, it has been reported that "natural immune" which is likewise one immunoreaction recognizes a difference between self-cells and foreign matters (for example, a constituent of bacteria) and an immunoreaction occurs on the basis of this difference (for example, Werling, D., and Jungi, W. T., Vet. Immunol. Immunopathol. 91: 1-12, 2003 and Poltorak, A., et al., Science, 282: 2085-2088, 1998). As one of such foreign matters, there is a CpG dinucleotide sequence (CpG sequence) contained in a bacterial DNA (for example, Hemmi, H., et al., Nature, 408: 740-745, 2000 and Kreig, A., et al., Nature, 374: 546-549, 1995).

In a chromosomal DNA of mammals, this CpG sequence is contained only at such a quite low frequency that its ratio is from approximately 1/50 to 1/60 a statistically expectable value, and the 5-position of most of cytosine bases of the CpG sequence is methylated with cytosine 5-methylase specific to the CpG sequence. Meanwhile, it has been reported that the ratio of the CpG sequence contained in a chromosomal DNA of bacteria (microbes) is approximately 1/16 which is almost equal to the statistically expectable value and it is a CpG sequence free from methylated cytosine like mammals (non-methylated CpG sequence) because there is no cytosine 5-methylase specific to CpG (for example, Bird, A. P., Trends Genet., 3:342-347, 1987). That is, the natural immune system of mammals is considered to be an immunoreaction that the system recognizes methylation or non-methylation of the CpG sequence to induce an inflammatory reaction (namely, enhancement of an immunoactivity) so as to be able to efficiently prevent the living body from invasion or infection of bacteria.

A natural immune-stimulating composition (gazette of JP-T-2003-527352 (the term "JP-T" as used herein means a published Japanese translation of a PCT patent application)), cancer immunotherapy (for example, Whitmore, M., Li, S., and Huang, L., Gene Ther., 6:1867-1875, 1999) and efficient DNA vaccines against infectious diseases (for example, gazette of JP-T-2002-511841, Brunner, C., et al., J. Immunol. 165: 6278-6286, 2000 and Kojima, and Y., et al., Vaccine, 20: 2857-2865, 2002) using enhancement of the immunoactivity with such a non-methylated CpG sequence have been reported and proposed to confirm its usefulness.

The difference in DNA sequence between microbes including bacteria and mammals is found in substances other than the CpG sequence. For example, N⁶- methyladenine (m⁶A) is mentioned. For example, adenine (A) of a GATC sequence in E. coli DNA is methylated with a DNA adenine methylase (Dam) which is an enzyme specifically acting on this adenine to give m⁶M. The inventors have considered that not only the non-methylated CpG sequence but also methylation of adenine in the GATC sequence (Gm⁶ATC sequence) functions as one mechanism of recognizing non-self foreign matters which mechanism has evolutionarily been stored by the natural immune. To study and elucidate what immunoactivity (biodefense reaction) is induced in the living body by this Gm⁶ATC sequence is considered to give quite an important knowledge in future when establishing gene therapy or the like using plasmids derived from bacteria such as E. coli.

Studies or reports on induction, promotion and enhancement of the immunoactivity (biodefense reaction) with a sequence containing a specific nucleic acid base except the non-methylated CpG sequence have been little made at present. As far as the inventors know, regarding induction and enhancement of the immunoactivity using the DNA containing the Gm⁶ATC sequence, for example, it has been proposed that a vaccine-like composition is formed with pathogenic bacteria such as salmonella attenuated by applying variation influencing DNA adenine methylase (Dam) and this is administered to the living body to increase an immunological effect and is used for prevention or therapy of infection of pathogenic bacteria (refer to gazette of JP-T-2002-536339).

However, since the vaccine-like composition described in gazette of JP-T-2002-536339 is made of pathogenic bacteria attenuated by applying variation of DNA adenine methylase, its effect is to increase the immunoactivity (biodefense reaction) against pathogenic bacteria as the origin of the vaccine-like composition. Accordingly, it is effective only against specific infectious diseases, and cannot enhance the immunoactivity of the overall living body to bring forth effects against various infectious diseases. That is, it is still unknown that a nucleic acid containing a specific nucleic acid base including a microbial nucleic acid-specific modified base, a plasmid having the nucleic acid or the like is administered to induce and enhance the immunoactivity.

Under these circumstances, the invention of this application has been made, and it aims to provide, upon solving the problems associated with the ordinary art, an immunopotentiator of mammals which comprises as an active ingredient a nucleic acid containing a special nucleic acid base or a plasmid having such a base, which is administered to mammals to be able to enhance the immunoactivity of the overall living body of mammals and which can be applied to cancer immunotherapy, gene therapy, DNA vaccines effective against various infectious diseases and the like, and a method for enhancing an immunoactivity using the same.

The invention of this application aims to provide cultured cells with an immunoactivity enhanced as model cells for studies on induction, enhancement or the like of an immunoactivity in vitro using the foregoing immunopotentiator. Further, it aims to provide non-human mammals with an immunoactivity enhanced as model animals for studies on an immunoactivity induced and enhanced in vivo using the immunopotentiator.

### Disclosure of the Invention

Embodiments of the present invention are defined in the claims.

In particular, the present invention provides an immunopotentiator for mammals, which comprises as an active ingredient a nucleic acid that is an oligonucleotide containing a microbial nucleic acid-specific modified base which is an N⁶-methyladenine base, a derivative thereof or a plasmid having said nucleic acid for use in enhancing an immunoactivity of mammals.

The invention also provides an immunopotentiator as described above, together with a composition comprising as an active ingredient a nucleic acid containing a microbial nucleic acid-specific non-methylated CpG sequence or a plasmid having the nucleic acid containing the microbial nucleic acid-specific non-methylated CpG sequence for use in enhancing an immunoactivity of mammals, wherein said immunopotentiator and said composition are simultaneously administered to mammals.

The invention also concerns a use of the immunopotentiator as described above, in the manufacture of a medicament for enhancing the immunoactivity of a mammal.

The invention also concerns a use of the immunopotentiator described above, together with a composition comprising as an active ingredient a nucleic acid containing a microbial nucleic acid-specific non-methylated CpG sequence or a plasmid having the nucleic acid containing the microbial nucleic acid-specific non-methylated CpG sequence in the manufacture of a medicament for enhancing the immunoactivity of a mammal, wherein said immunopotentiator and said composition are simultaneously administered to mammals.

The invention also concerns a process for producing an inflammatory cytokine, which comprises administering the immunopotentiator as described above to cultured cells to enhance an immunoactivity of the cultured cells and produce the inflammatory cytokine.

The invention also concerns a process for producing an inflammatory cytokine, which comprises simultaneously administering to cultured cells the immunopotentiator as described above, together with a composition comprising as an active ingredient a nucleic acid containing a microbial nucleic acid-specific non-methylated CpG sequence or a plasmid having the nucleic acid containing the microbial nucleic acid-specific non-methylated CpG sequence to further enhance an immunoactivity and produce the inflammatory cytokine.

The invention also provides an immunopotentiator for mammals, which comprises as an active ingredient a nucleic acid containing a microbial nucleic acid-specific modified base which is an N⁶-methyladenine base wherein said nucleic acid has a base sequence of SEQ ID NO: 4, a derivative thereof or a plasmid having said nucleic acid.

Also described herein are the following (1) to (19) as a means for solving the foregoing problems.
(1) An immunopotentiator for mammals, which comprises as an active ingredient a nucleic acid containing a special nucleic acid base, a derivative thereof or a plasmid having the nucleic acid containing the special nucleic acid base.
(2) The immunopotentiator of (1), wherein the special nucleic acid base is at least one selected from the group consisting of 8-oxoguanine, 8-oxoadenine, 2-oxoadenine, 5-hydroxyuracil, 5-formyluracil, 5-formylcytosine, 8-nitroguanine, thymine glycol, cytosine glycol, hypoxanthine, oxanine, pyrimidine dimmer, O⁶-methylguanine and O⁴-methylthymine.
(3) The immunopotentiator of (1), wherein the special nucleic acid base is a microbial nucleic acid-specific modified base.
(4) The immunopotentiator of (3), wherein the microbial nucleic acid-specific modified base is at least one selected from the group consisting of N⁶-methyladenine, 5-hydroxymethyluracil and 5-hydroxymethylcytosine.
(5) The immunopotentiator of (3), wherein the nucleic acid containing the microbial nucleic acid-specific modified base is a nucleic acid having a base sequence of SEQ ID NO: 4.
(6) The immunopotentiator of any of (1) to (5), which further comprises as an active ingredient a nucleic acid containing a microbial nucleic acid-specific non-methylated CpG sequence or a plasmid having the nucleic acid containing the microbial nucleic acid-specific non-methylated CpG sequence.
(7) The immunopotentiator of (6), wherein the nucleic acid containing the microbial nucleic acid-specific non-methylated CpG sequence is a nucleic acid having the base sequence of SEQ ID NO: 2.
(8) The immunopotentiator of any of (3) to (7), wherein the microbe is a virus or a bacterium.
(9) The immunopotentiator of (8), wherein the bacterium is Escherichia coli.
(10) A process for producing an inflammatory cytokine, which comprises administering the immunopotentiator of any of (1) to (9) to cultured cells to enhance an immunoactivity of the cultured cells and produce the inflammatory cytokine.
(11) A process for producing an inflammatory cytokine, which comprises simultaneously administering to cultured cells the immunopotentiator of any of (1), (2), (3), (4), (5), (8) and (9) together with a composition comprising as an active ingredient a nucleic acid containing a microbial nucleic acid-specific non-methylated CpG sequence or a plasmid having the nucleic acid containing the microbial nucleic acid-specific non-methylated CpG sequence to further enhance an immunoactivity and produce the inflammatory cytokine.
(12) Cultured cells producing an inflammatory cytokine, to which the immunopotentiator of any of (1) to (9) is administered to enhance an immunoactivity.
(13) Cultured cells producing an inflammatory cytokine, to which the immunopotentiator of any of (1), (2), (3), (4), (5), (8) and (9) together with a composition comprising as an active ingredient a nucleic acid containing a microbial nucleic acid-specific non-methylated CpG sequence or a plasmid having the nucleic acid containing the microbial nucleic acid-specific non-methylated CpG sequence are simultaneously administered to further enhance an immunoactivity.
(14) The cultured cells of (12) or (13), which are derived from mammals including humans.
(15) A method for enhancing an immunoactivity of mammals, which comprises administering to mammals the immunopotentiator of any of (1) to (9) to enhance an immunoactivity of mammals.
(16) A method for enhancing an immunoactivity of mammals, which comprises simultaneously administering to mammals the immunopotentiator of any of (1), (2), (3), (4), (5), (8) and (9) together with a composition comprising as an active ingredient a nucleic acid containing a microbial nucleic acid-specific non-methylated CpG sequence or a plasmid having the nucleic acid containing the microbial nucleic acid-specific non-methylated CpG sequence to further enhance an immunoactivity of mammals.
(17) Non-human mammals to which the immunopotentiator of any of (1) to (9) is administered to enhance an immunoactivity.
(18) Non-human mammals to which the immunopotentiator of any of (1), (2), (3), (4), (5), (8) and (9) together with a composition comprising as an active ingredient a nucleic acid containing a microbial nucleic acid-specific non-methylated CpG sequence or a plasmid having the nucleic acid containing the microbial nucleic acid-specific non-methylated CpG sequence are simultaneously administered to further enhance an immunoactivity.
(19) The non-human mammals of (17) or (18), which are mice.

By the invention of this application and the subject matter recited in (1) to (19), the immunopotentiator is provided which comprises as an active ingredient the nucleic acid containing the special nucleic acid base or the plasmid having the nucleic acid, which is administered to mammals to be able to enhance the immunoactivity of the overall living body of mammals and which can be applied to cancer immunotherapy, gene therapy, DNA vaccines effective against various infectious diseases and the like.

The method for enhancing the immunoactivity which can enhance the immunoactivity of the overall living body of mammals using the foregoing immunopotentiator is further provided.

Also described are the cultured cells which can efficiently produce the inflammatory cytokine using the foregoing immunopotentiator and the process for producing the inflammatory cytokine using the cells are still further provided.

Also described are the non-human mammals with the immunoactivity enhanced which are moreover provided as in vivo model animals for studies on the immunoactivity which is induced or enhanced in vivo by the foregoing immunopotentiator.

### Brief Description of the Drawings

Fig. 1 is a graph showing results of measuring IL-6 by single administration of a Gm⁶ATC sequence or a non-methylated CpG sequence to mice.
Fig. 2 is a graph showing results of measuring IL-12 by single administration of a Gm⁶ATC sequence or a non-methylated CpG sequence to mice.
Fig. 3 is a graph showing results of measuring TNF-α by single administration of a Gm⁶ATC sequence or a non-methylated CpG sequence to mice.
Fig. 4 is a graph showing results of measuring IL-6 by simultaneous administration of a Gm⁶ATC sequence and a non-methylated CpG sequence to mice.
Fig. 5 is a graph showing results of measuring IL-12 by simultaneous administration of a Gm⁶ATC sequence and a non-methylated CpG sequence to mice.
Fig. 6 is a graph showing results of measuring TNF-α by simultaneous administration of a Gm⁶ATC sequence and a non-methylated CpG sequence to mice.
Fig. 7 is a graph showing results of measuring IL-6 and IL-12 by administration of a Gm⁶ATC sequence-containing plasmid to mice.

### Best Mode for Carrying Out the Invention

The invention of this application has the foregoing characteristics, and the embodiments thereof are described in detail below.

The immunopotentiator of mammals in this application is characterized by comprising as an active ingredient the nucleic acid containing the special nucleic acid base, a derivative thereof or any plasmid having the nucleic acid containing the special nucleic acid base.

The "special nucleic acid base" in the invention of this application means a special base as an ingredient of a nucleic acid, such as a special base as a DNA ingredient or a special base as an RNA ingredient. Specifically, it is a base except adenine, guanine, cytosine, thymine and uracil, such as a microbial nucleic acid-specific modified base. Through a nucleic acid such as a DNA or RNA having the special base, an immune system of mammals can distinguish and recognize non-self to enhance and promote the immunoactivity. More specific examples are bases which are subjected to various modifications such as hydroxylation and methylation. Examples thereof include 8-oxoguanine, 8-oxoadenine, 2-oxoadenine, 5-hydroxyuracil, 5-formyluracil, 5-formylcytosine, 8-nitroguanine, thymine glycol, cytosine glycol, hypoxanthine, oxanine, pyrimidine dimer, O⁶-methylguanine, O⁴-methylthymine and the like. These may be used either singly or in combination of two or more.

Further, the "plasmid" may express or may not express each gene that the plasmid carries in cells of mammals, and its type is not particularly limited. For example, pQBI63 and pcDNA are available.

The "microbial nucleic acid-specific modified base" means the foregoing special base which is carried specifically in microbes. Through the microbial nucleic acid-specific modified base, the immune system of mammals can distinguish between self (mammals) and non-self (microbes such as bacteria) to enhance and promote the immunoactivity. The type of the microbial nucleic acid-specific modified base of the invention of this application is N⁶-methyladenine. Other examples thereof can include 5-hydroxymethyluracil, 5-hydroxymethylcytosine and the like. These may be used either singly or in combination of two or more.

Especially when the microbial nucleic acid-specific modified base is N6-methyladenine, the base sequence containing the base to be modified in this N⁶-methyladenine is preferably a GATC sequence for preferentially selecting the "GATC sequence" to methylate the N-6 position of adenine (A). Specifically, a base sequence indicated in SEQ ID NO: 4 is more preferable.

Regarding the microbial nucleic acid-specific modified base, a nucleic acid such as a natural DNA or RNA which is isolated from microbes such as bacteria including Escherichia coli and viruses by a known method can be used. For example, a nucleic acid (artificial oligonucleotide) formed by artificially adding a modified base in a known manner may be used (for example, Cowdery, J. S., et al., J. Immunol., 156, 4570-4575, 1996).

In the invention of this application, the derivative of the nucleic acid containing the special nucleic acid base may be used. This "derivative" is a substance whose phosphoric acid moiety or sugar moiety is modified in using a chemical synthetic product, a substance with a structure other than a base moiety changed or the like. For example, a phosphorothioate-modified substance, a 2'-O-methyl RNA or a peptide nucleic acid (PNA) can be used.

As noted above, in mammals, the nucleic acid is almost free from the CpG dinucleotide sequence (CpG sequence). Or even though it has the CPG dinucleotide sequence, the 5-position is methylated with the CpG sequence-specific cytosine 5-methylase. Accordingly, the bioimmune system recognizes the difference to enhance and induce the immunoactivity. Thus, the immunopotentiator in the invention of this application can more enhance the immunoactivity of mammals by further comprising as an active ingredient the nucleic acid containing the microbial nucleic acid-specific non-methylated CpG sequence or the plasmid having the nucleic acid containing the microbial nucleic acid-specific non-methylated CpG sequence. Specifically, the nucleic acid having the non-methylated CpG sequence is preferably a base sequence indicated in, for example, SEQ ID NO: 2.

The "microbe" in the invention of this application is a virus or a bacterium, and Escherichia coli whose handling method or knowledge has been abundantly accumulated is especially preferable as the bacterium.

Also described are cultured cells. The immunopotentiators described above are administered to the cultured cells by a known method such as a method of administration along with a cationic lipid, a microinjection method or an electroporation method so as to be able to enhance the immunoactivity of the cultured cells and efficiently produce the inflammatory cytokine. The cultured cells are available as model cells for experiment on the in vitro immunoactivity. The inflammatory cytokine can efficiently be produced and obtained by using the cultured cells. The resulting inflammatory cytokine can be used in various applications such as therapeutic agents by extraction, purification and the like in a usual manner.

For further enhancing the immunoactivity, it is also possible to simultaneously administer to any cultured cells a combination of the foregoing immunopotentiator and a composition comprising the nucleic acid containing the microbial nucleic acid-specific non-methylated CpG sequence or the plasmid having the nucleic acid containing the microbial nucleic acid-specific non-methylated CpG sequence as the active ingredient.

The type, the origin and the like of the "cultured cells" are not particularly limited. For example, tissues or cells of individual organisms are available. Specific examples thereof include plant cells, insect cells, mammalian cells and the like. Various constituents of these tissues are also available. In particular, since the invention of this application is to enhance and promote the immune system of mammals, the cultured cells used are preferably derived from mammals including humans. For example, cultured cells derived from humans, monkeys, horses, cattle, pigs, sheep, mice, rats , rabbits and the like can be used.

Also described is a method for enhancing the immunoactivity of individual mammals in which the immunoactivity of mammals can be enhanced by administering the above-described immunopotentiators to mammals.

When the immunopotentiator of the invention of this application is administered to mammals, it can be administered in the form of a nucleic acid. It is preferable that the immunopotentiator is administered by containing therein various pharmacological ingredients according to the dosage form to enhance the immunoactivity. The "pharmacological ingredients" first mean various carriers which are used in ordinary preparation of agents. The carriers can properly be selected from the wide range according to the type of the disease and the dosage form of the agent. A unit dosage form capable of oral administration or administration by injection is preferable. Especially, in the administration by injection, local injection, intraperitoneal administration, selective intravenous injection, intravenous injection, subcutaneous injection, organ infusion liquid injection and the like can be employed.

Oral liquid preparations such as suspensions and syrups can be prepared using water, sugars such as sucrose, sorbitol and fructose, glycols such as polyethylene glycol, oils such as sesame oil and soybean oil, preservatives such as alkyl p-hydroxybenzoate, flavors such as a strawberry flavor and peppermint, and the like.

Powders, pills, capsules and tablets can be prepared using excipients such as lactose, glucose, sucrose and mannitol, disintegrants such as starch and sodium alginate, lubricants such as magnesium stearate and talc, binders such as polyvinyl alcohol, hydroxypropyl cellulose and gelatin, surfactants such as fatty acid ester, plasticizers such as glycerin, and the like. When preparing tablets and capsules, solid pharmaceutical carriers are used.

The injection solution can be prepared using a carrier made of a salt solution, a glucose solution, a mixture of a salt solution and a glucose solution, various buffer solutions or the like. The injection solution may be prepared in a powdery state and mixed with the liquid carrier when used.

Attention has to be drawn to the fact that the dose of the immunopotentiator in the invention of this application varies with the weight of mammals as an administration subject, the condition of diseases, the administration route and the like.

The second pharmacological ingredients are ingredients by which the immunopotentiator is formulated in a dosage form capable of introduction into cells. For example, a composition can be formed by mixing the nucleic acid containing the microbial nucleic acid-specific modified base as the active ingredient of the immunopotentiator or the plasmid containing the same with a pharmacologically acceptable solution without changing the structure or the function of the nucleic acid or plasmid. Such a composition can also be introduced into target cells by a method of introduction into cells via microinjection, a method of introduction into cells using lipids (for example, BioPORTER (Gene Therapy Systems, U.S.A.)) or peptide reagents (for example, Chariot (Active Motif, U.S.A.)), or through a gene gun by adhesion to gold particles.

As described above, the immune system of mammals distinguishes between the methylated CpG sequence of mammals and the microbe-specific non-methylated CpG sequence to enhance and induce the immunoactivity of mammals. Accordingly, the immunoactivity can be more enhanced by simultaneously administering to mammals the foregoing immunopotentiator and the composition comprising as the active ingredient the nucleic acid containing the microbial nucleic acid-specific non-methylated CpG sequence or the plasmid having the nucleic acid containing the microbial nucleic acid-specific non-methylated CpG sequence in combination.

Examples of the "mammals" in the invention of this application can include mammals such as humans, monkeys, horses, cattle, pigs, sheep, mice, rats, rabbits and the like, and the immunopotentiator can be administered thereto.

Thus, applications to cancer immunotherapy, gene therapy, development of DNA vaccines effective against various infectious diseases, and the like can be expected by enhancing the immunoactivity of mammals according to the immunopotentiator of mammals and the method for enhancing the immunoactivity using the same which are provided by the invention of this application.

Also described are model animals for study on the immunoactivity induced or enhanced in vivo by the nucleic acid containing the specific nucleic acid base, the derivative thereof, the plasmid containing the nucleic acid or the like. In this case, it is important that the mammals are non-human mammals except humans. Examples thereof can include monkeys, horses, cattle, pigs, sheep, mice, rats, rabbits and the like. Especially, mice for which an abundant knowledge or the like has been accumulated and which have been used in many laboratories as experimental model animals are preferable in view of its easy handling. The experimental model animals can contribute to elucidation of a mechanism of inflammation induction (immunoactivity) with a specific nucleic acid base (modified base) such as a Gm⁶ATC sequence.

The strength of the inflammatory reaction induced by preparing a synthetic nucleic acid (oligonucleotide) containing or not containing m⁶A and a plasmid containing the same and administering each thereof to a Balb/c mouse is examined using inflammatory cytokines as an index, and is demonstrated as Example to illustrate the invention of this application more specifically. Of course, the invention of this application is not limited by the following Example.

### Example

### 1. Induction of an inflammatory reaction by a Gm⁶ATC sequence

### (1) Synthesis of a Gm⁶ATC sequence and a non-methylated CPG sequence

Regarding a phosphothioate-stabilized oligonucleotide (ODN) shown in Table 1, according to a known method (for example, Cowdery, J.S., et al., J. Immunol., 156, 4570-4575, 1996), four types of sequences, a GATC sequence, a Gm⁶ATC sequence, a non-methylated CpG sequence and a methylated CpG sequence were synthesized (Sigma Gonosys Japan). Each synthetic ODN was prepared at a concentration of 1 nmol/µl by being dissolved in Endotoxine free TE buffer (QIAGEN). Subsequently, Limulus Amoebocyte Lysate assay (LAL test; PYROGENT, BioWhittaker) was conducted to confirm that the content of the endotoxin in this synthetic ODN solution was 0.006 EU/ml or less.

**Table 1**

| ODN | Motif | Sequence (5'-3') |
|---|---|---|
| GpC-ODN1720 | non CpG | TCC ATG AGC TTC CTG ATG CT |
| CpG-ODN1668 | CpG | TCC ATG ACG TTC CTG ATG CT |
| GATC-dA | non-m⁶A | TCC ATG ATC TTC CTG ATG CT |
| GATC-m⁶A | m⁶A | TCC ATG m⁶ATC TTC CTG ATG CT |

### (2) Administration of a Gm⁶ATC sequence and a non-methylated CpG sequence

### (i) Single administration of a Gm⁶ATC sequence or a non-methylated CpG sequence

10 nmol of each of GATC-dA (sequence containing a GATC sequence), GATC-m⁶A (sequence containing a Gm⁶ATC sequence), CpG-ODN1668 (sequence containing a non-methylated CpG sequence) and GpC-ODN 1720 (sequence containing a methylated CpG sequence) shown in Table 1 was collected, and dissolved in 400 µl of a physiological saline solution. The resulting solution was intraperitoneally administered to a Ba1b/c mouse (male, 6 weeks old; Sankyo Labo-Service). Two hours later, concentrations of inflammatory cytokines (IL-6, IL-12 and TNF-α) in the serum were measured by an enzyme-linked immunosorbent assay (ELISA; AN'ALIZA, Genzyme TECHNE corp.).

Two hours after the administration, the blood was collected from the heart of the mouse, and allowed to stand overnight at 4°C. The serum was collected therefrom by centrifugation (20,000 g, 20 minutes, 4°C).

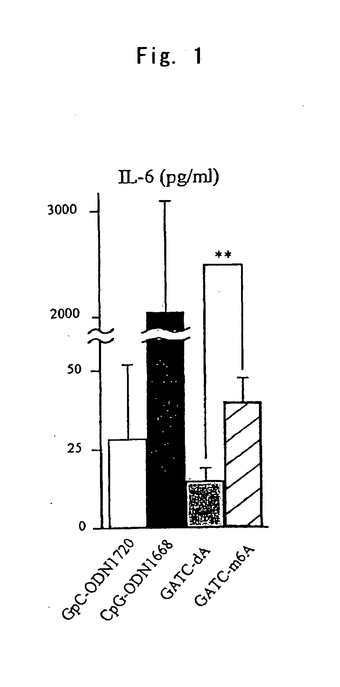

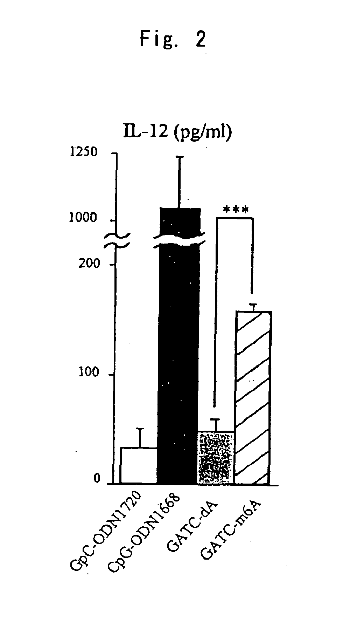

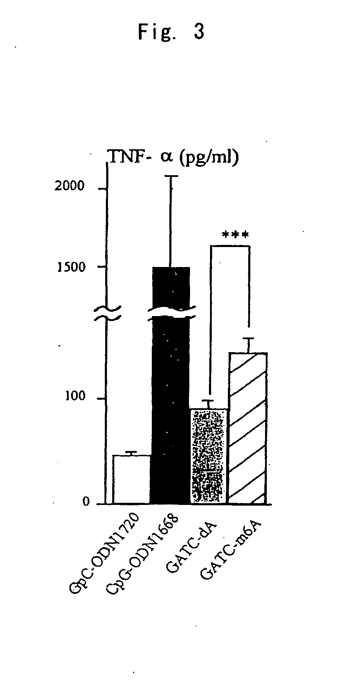

The results are as shown in Figs. 1 to 3. Fig. 1 shows a measured value (pg/ml) of IL-6, Fig. 2 a measured value (pg/ml) of IL-12, and Fig. 3 a measured value (pg/ml) of TNF-α. By the way, the results shown in Figs. 1 to 3 are expressed in terms of a mean value ± standard deviation. In the graphs, two asterisks indicate "P<0.01", and three asterisks "P<0.005 (n=3 (IL-12), 4 (IL-6, TNF-α)))".

Measured values in GpC-ODN1720 are IL-6:28 pg/ml, IL-12:33 pg/ml and TNF-α:47 pg/ml. Inflammatory cytokines were little induced. However, in case of administering CpG-ODN1668, very strong inflammatory cytokines were induced, and measured values thereof were IL-6:2.1 ng/ml, IL-12:1.0 ng/ml and TNF-α:1.5 ng/ml.

Meanwhile, with respect to the comparison in induction of inflammatory cytokines between GATC-dA and GATC-m⁶A, it was confirmed that the cytokines were significantly induced by administration of GATC-m⁶A in comparison to the administration of GATC-dA, though the induction by administration of GATC-m⁶A was slightly lower than the induction by administration of CpG-ODN1668. That is, the measured values in GATC-m⁶A were IL-6:39 pg/ml, IL-12:160 pg/ml and TNF-α:150 pg/ml, and the measured values in GATC-dA were IL-6:14 pg/ml, IL-12:48 pg/ml and TNF-α:92 pg/ml. This indicates that the inflammatory cytokines are specifically induced by methylation of adenine (m⁶A). Further, the immune inducibility of the non-methylated CpG sequence differs depending on the sequences before and after the very sequence (Kreig, A., et al., Nature, 374: 546-549, 1995 and the like). Consequently, the strong immune induction effect can be expected even via the single administration of the Gm⁶ATC sequence by optimizing the sequences before and after the very sequence.

### (ii) Simultaneous administration of a Gm⁶ATC sequence and a non-methylated CpG sequence

A microbial DNA (nucleic acid) contains not only m⁶A but also a non-methylated CpG sequence. For example, in gene therapy of a peripheral arterial disease with a non-viral vector, a plasmid containing a Gm⁶ATC sequence and a non-methylated CpG sequence is used (for example, Baumgartner, I., et al., Circulation, 97, pp. 1114-1123, 1998).

Accordingly, the Gm⁶ATC sequence and CpG-ODN1668 containing the non-methylated sequence were simultaneously administered to a mouse to confirm an effect of inducting inflammatory cytokines. Regarding the doses of Gm⁶ATC, GATC-dA and CpG-ODN1668, 5 nmol of CpG-ODN1668 was mixed with an equimolar amount of Gm⁶ATC or GATC-dA, and the mixture was dissolved in 400 µl of a physiological saline solution. The resulting solution was administered to a mouse. Two hours later, the inflammatory cytokines in the serum of the mouse were measured by ELISA to confirm induction amounts.

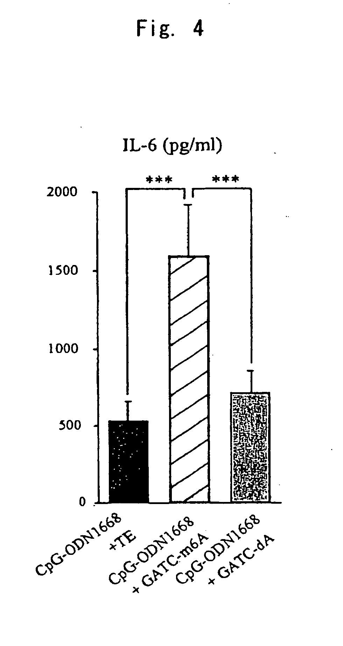

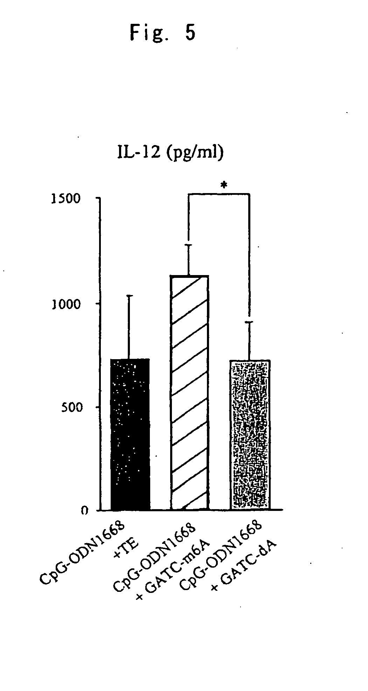

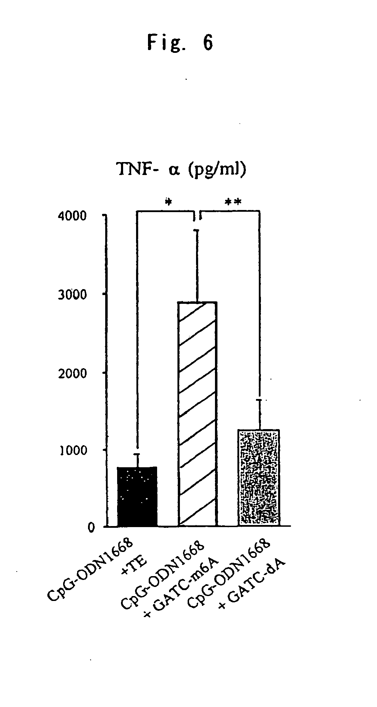

The results are as shown in Figs. 4 to 6. Fig. 4 shows a measured value (pg/ml) of IL-6, Fig. 5 a measured value (pg/ml) of IL-12 and Fig. 6 a measured value (pg/ml) of TNF-α. The results shown in Figs. 4 to 6 are expressed in terms of a mean value ± standard deviation (n=4). In the graphs, one asterisk indicates "P<0.05", two asterisks "P<0.01" and three asterisks "P<0.005".

That is, in the single administration of CpG-ODN1668, the measured values were IL-6:530 pg/ml, IL-12:730 pg/ml and TNF-α:760 pg/ml. In the simultaneous administration of CpG-ODN1668 and GATC-dA, the measured values were IL-6:710 pg/ml, IL-12:720 pg/ml and TNF-α:1200 pg/ml. In the simultaneous administration of CpG-ODN1668 and Gm⁶ATC, the measured values were IL-6:1600 pg/ml, IL-12:1100 pg/ml and TNF-α:2900 pg/ml, and it could be confirmed that the cytokines were induced at remarkably high concentrations. The concentrations were 2 to 3 times those in the single administration of CpG-ODN1668 and the simultaneous administration of CpG-ODN1668 and GATC-dA. It was confirmed that when m⁶A and the non-methylated CpG sequence coexisted, the inflammatory cytokines were induced and enhanced quite strongly. 2. Induction of an inflammatory reaction by a plasmid having incorporated therein a Gm⁶ATC sequence-containing nucleic acid (oligonucleotide) (1) Preparation of a plasmid

Using a plasmid employed in actual gene therapy, an inflammatory reaction induced by a Gm⁶ATC sequence contained in the plasmid was examined.

As the plasmid, plasmid pQBI63 in which gene expression is not conducted in cells of mammals was used. As host bacteria, Escherichia coli DH5α (dam⁺) having a methylase activity and Escherichia coli SCS110 (dam⁻) having no methylase activity were prepared. Plasmid pQBI63 was introduced into each Escherichia coli by a known method (for example, a heat shock method or an electroporation method). Purification was conducted using Endo free plasmid mega kit (QIAGEN).

The plasmid prepared by introducing pQBI63 into DH5α (dam⁺) was designated pQBI63/DH5α, and the plasmid prepared by introducing pQBI63 into SCS110 (dam-) was designated pQBI63/SCS110. Plasmids pQBI63/DH5α and pQBI63/SCS110 after preparation were collected by low-melting agarose electrophoresis. Subsequently, they were purified using QIA-tip 100 (QIAGEN) or Micropure-EZ (Millipore) and the LAL test (PYROGENT, BioWhittaker) was conducted as in 1.(1) to confirm that the content of endotoxin was 0.006 EU/ml or less.

### (2) Methylation reaction in vitro

90 µg of pQBI63/SCS110 obtained in (1) was collected, and charged into 120 µl of a methylase buffer (50 mM Tris-HCl, 10 mM EDTA, 5 mM 2-mercaptoethanol (pH 7.5)). The reaction was conducted overnight at 37°C using 19.2 U of Dam methylase (New England BioLabos Inc.). At this time, S-adenosylmethionine (SAM; New England BioLabos Inc.) as a methyl group donor was added at a concentration of 80 µM to methylate adenine. This plasmid was designated pQBI63/SAM+. A mock-methylated plasmid without addition thereof was designated pQBI63/SAM-. After completion of the reaction, pQBI63/SAM+ and pQBI63/SAM- were purified using Endo free plasmid mega kit (QIAGNE) as in 2.(1) to remove endotoxin and prevent incorporation.

### (3) Administration of plasmids

The inventors have considered that a method in which a plasmid prepared from Escherichia coli is administered along with a cationic lipid in gene therapy using a non-viral vector will be generally used in future. Thus, a working example in which the plasmid in the invention of this application was administered along with a cationic lipid was shown as an example.

Each of pQBI63/DH5α and pQBI63/SCS110 prepared in 2. (1), or each of pQBI63/SAM+ and pQBI63/SAM- prepared in 2. (2) was formed into a complex with Lipofectin (Invitrogen) as a cationic lipid (cationic liposome), and the complex was administered into a mouse through the tail vein. Four hours after the administration, the serum was collected as in 1. (2)(i), and the concentrations (pg/ml) of inflammatory cytokines, IL-6 and IL-12 in the serum were measured and quantified by ELISA.

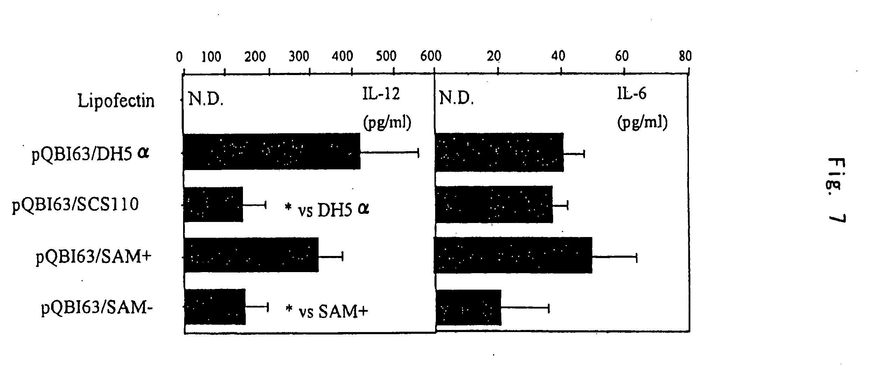

The results are as shown in Fig. 7, and expressed in terms of a mean value ± standard deviation. One asterisk indicates "P<0.05 (n=3)".

First, regarding the results of measuring IL-12, IL-12 was induced at a concentration of up to 420 pg/ml with pQBI63/DH5α, whereas the administration of pQBI63/SCS110 reduced the induction of IL-12 to 140 pg/ml.

Subsequently, pQBI63/SAM+ obtained by Dam-treatment of pQBI63/SCS110 in the presence of S-adenosylmethionine to methylate adenine was administered to induce 320 pg/ml of IL-12 which was close to the value in pQBI63/DH5α. pQBI63/SAM- obtained by Dam-treatment in the absence of S-adenosylmethionine induced 140 pg/ml of IL-12 which was the same as the value in pQBI63/SCS110.

In the results of measuring IL-6, it was confirmed that the induction of IL-6 was not so strong as the induction of IL-12 but the way of the induction of IL-6 was approximately the same as that of the induction of IL-12. That is, it is indicated that the inflammatory reaction is induced and enhanced by the Gm⁶ATC sequence.

Incidentally, the concentrations of IL-12 and IL-6 in the serum at the time of the single administration of Lipofectin as a control were under the detection limit value (<7.8 pg/ml).

### Industrial Applicability

As has been thus far described in detail, this application provides the immunopotentiator using the nucleic acid containing the special nucleic acid base or the plasmid having the nucleic acid containing the special nucleic acid base, and the method for enhancing the immunoactivity using the same, and applications thereof to elucidation of the mechanism of the immunoactivity, cancer therapy, development of DNA vaccines against various infectious diseases and the like can be expected.

## Claims

1. An immunopotentiator for mammals, which comprises as an active ingredient a nucleic acid that is an oligonucleotide containing a microbial nucleic acid-specific modified base which is an N⁶-methyladenine base, a derivative thereof or a plasmid having said nucleic acid for use in enhancing an immunoactivity of mammals.

2. The immunopotentiator for use as claimed in claim 1, wherein the nucleic acid comprises a GATC sequence in which the A is an N⁶-methyladenine base.

3. The immunopotentiator for use as claimed in claim 1 or claim 2, wherein the nucleic acid is a nucleic acid having a base sequence of SEQ ID NO: 4.

4. The immunopotentiator for use as claimed in any of claims 1 to 3, which further comprises as an active ingredient a nucleic acid containing a microbial nucleic acid-specific non-methylated CpG sequence or a plasmid having the nucleic acid containing the microbial nucleic acid-specific non-methylated CpG sequence.

5. The immunopotentiator for use as claimed in claim 4, wherein the nucleic acid containing the microbial nucleic acid-specific non-methylated CpG sequence is a nucleic acid having the base sequence of SEQ ID NO: 2.

6. The immunopotentiator for use as claimed in any one of claims 1 to 5, wherein the microbe is a virus or a bacterium.

7. The immunopotentiator for use as claimed in claim 6, wherein the bacterium is Escherichia coli.

8. An immunopotentiator as defined in any of claims 1, 2, 3, 6 or 7, together with a composition comprising as an active ingredient a nucleic acid containing a microbial nucleic acid-specific non-methylated CpG sequence or a plasmid having the nucleic acid containing the microbial nucleic acid-specific non-methylated CpG sequence for use in enhancing an immunoactivity of mammals, wherein said immunopotentiator and said composition are simultaneously administered to mammals.

9. Use of an immunopotentiator as defined in any one of claims 1 to 7 in the manufacture of a medicament for enhancing the immunoactivity of a mammal.

10. Use of an immunopotentiator as defined in any of claims 1, 2, 3, 6 or 7, together with a composition comprising as an active ingredient a nucleic acid containing a microbial nucleic acid-specific non-methylated CpG sequence or a plasmid having the nucleic acid containing the microbial nucleic acid-specific non-methylated CpG sequence in the manufacture of a medicament for enhancing the immunoactivity of a mammal, wherein said immunopotentiator and said composition are simultaneously administered to mammals.

11. A process for producing an inflammatory cytokine, which comprises administering the immunopotentiator as defined in any of claims 1 to 7 to cultured cells to enhance an immunoactivity of the cultured cells and produce the inflammatory cytokine.

12. A process for producing an inflammatory cytokine, which comprises simultaneously administering to cultured cells the immunopotentiator as defined in any of claims 1, 2, 3, 6 or 7, together with a composition comprising as an active ingredient a nucleic acid containing a microbial nucleic acid-specific non-methylated CpG sequence or a plasmid having the nucleic acid containing the microbial nucleic acid-specific non-methylated CpG sequence to further enhance an immunoactivity and produce the inflammatory cytokine.

13. An immunopotentiator for mammals, which comprises as an active ingredient a nucleic acid containing a microbial nucleic acid-specific modified base which is an N⁶-methyladenine base wherein said nucleic acid has a base sequence of SEQ ID NO: 4, a derivative thereof or a plasmid having said nucleic acid.

14. The immunopotentiator of claim 13, which further comprises the features as defined in any one of claims 4 to 7.

## Patentansprüche

1. Immunpotentiator für Säugetiere, welcher als einen Wirkstoff eine Nukleinsäure, die ein Oligonukleotid ist, das eine nukleinsäurespezifische modifizierte Base eines Mikroorganismus enthält, welche eine N⁶-Methyladeninbase ist, ein Derivat davon oder ein Plasmid umfasst, welches die Nukleinsäure aufweist, zur Verwendung bei der Verstärkung einer Immunaktivität von Säugetieren.

2. Immunpotentiator zur Verwendung nach Anspruch 1, wobei die Nukleinsäure eine GATC-Sequenz umfasst, in welcher A eine N⁶-Methyladeninbase ist.

3. Immunpotentiator zur Verwendung nach Anspruch 1 oder Anspruch 2, wobei die Nukleinsäure eine Nukleinsäure ist, die eine Basensequenz von SEQ. ID. NR.: 4 aufweist.

4. Immunpotentiator zur Verwendung nach einem der Ansprüche 1 bis 3, welcher des Weiteren eine Nukleinsäure, die eine nukleinsäurespezifische nichtmethylierte CpG-Sequenz eines Mikroorganismus enthält, oder ein Plasmid mit der Nukleinsäure, die die nukleinsäurespezifische nichtmethylierte CpG-Sequenz eines Mikroorganismus enthält, als einen Wirkstoff umfasst.

5. Immunpotentiator zur Verwendung nach Anspruch 4, wobei die Nukleinsäure, die die nukleinsäurespezifische nichtmethylierte CpG-Sequenz eines Mikroorganismus enthält, eine Nukleinsäure ist, die die Basensequenz von SEQ. ID. NR.: 2 aufweist.

6. Immunpotentiator zur Verwendung nach einem der Ansprüche 1 bis 5, wobei der Mikroorganismus ein Virus oder ein Bakterium ist.

7. Immunpotentiator zur Verwendung nach Anspruch 6, wobei das Bakterium Escherichia coli ist.

8. Immunpotentiator, wie in einem der Ansprüche 1, 2, 3, 6 oder 7 definiert, zusammen mit einer Zusammensetzung, umfassend als einen Wirkstoff eine Nukleinsäure, welche eine nukleinsäurespezifische nichtmethylierte CpG-Sequenz eines Mikroorganismus enthält, oder ein Plasmid, das die Nukleinsäure aufweist, welche die nukleinsäurespezifische nichtmethylierte CpG-Sequenz eines Mikroorganismus enthält, zur Verwendung bei der Verstärkung einer Immunaktivität von Säugetieren, wobei der Immunpotentiator und die Zusammensetzung gleichzeitig an Säugetiere verabreicht werden.

9. Verwendung eines Immunpotentiators, wie in einem der Ansprüche 1 bis 7 definiert, bei der Herstellung eines Medikaments zur Verstärkung der Immunaktivität eines Säugetiers.

10. Verwendung eines Immunpotentiators, wie in einem der Ansprüche 1, 2, 3, 6 oder 7 definiert, zusammen mit einer Zusammensetzung, umfassend als einen Wirkstoff eine Nukleinsäure, welche eine nukleinsäurespezifische nichtmethylierte CpG-Sequenz eines Mikroorganismus enthält, oder ein Plasmid, das die Nukleinsäure aufweist, welche die nukleinsäurespezifische nichtmethylierte CpG-Sequenz eines Mikroorganismus enthält, bei der Herstellung eines Medikaments zur Verstärkung der Immunaktivität eines Säugetiers, wobei der Immunpotentiator und die Zusammensetzung gleichzeitig an Säugetiere verabreicht werden.

11. Verfahren zur Herstellung eines entzündlichen Zytokins, welches die Verabreichung des Immunpotentiators, wie in einem der Ansprüche 1 bis 7 definiert, an kultivierte Zellen umfasst, um eine Immunaktivität der kultivierten Zellen zu verstärken und das entzündliche Zytokin herzustellen.

12. Verfahren zur Herstellung eines entzündlichen Zytokins, welches die gleichzeitige Verabreichung des Immunpotentiators, wie in einem der Ansprüche 1, 2, 3, 6 oder 7 definiert, zusammen mit einer Zusammensetzung, die eine Nukleinsäure, welche eine nukleinsäurespezifische nichtmethylierte CpG-Sequenz eines Mikroorganismus enthält, oder ein Plasmid, das die Nukleinsäure aufweist, welche die nukleinsäurespezifische nichtmethylierte CpG-Sequenz eines Mikroorganismus enthält, als einen Wirkstoff umfasst, an kultivierte Zellen umfasst, um eine Immunaktivität weiter zu verstärken und das entzündliche Zytokin herzustellen.

13. Immunpotentiator für Säugetiere, welcher eine Nukleinsäure, die eine nukleinsäurespezifische modifizierte Base eines Mikroorganismus enthält, welche eine N⁶-Methyladeninbase ist, wobei die Nukleinsäure eine Basensequenz von SEQ. ID. NR.: 4 aufweist, ein Derivat davon oder ein Plasmid, welches die Nukleinsäure enthält, als einen Wirkstoff umfasst.

14. Immunpotentiator nach Anspruch 13, welcher des Weiteren die Merkmale, wie in einem der Ansprüche 4 bis 7 definiert, umfasst.

## Revendications

1. Immunopotentialisateur pour mammifères, qui comprend en tant que principe actif un acide nucléique qui est un oligonucléotide contenant une base modifiée spécifique de l'acide nucléique microbien qui est une base N⁶-méthyladénine, un dérivé de celui-ci ou un plasmide comportant ledit acide nucléique pour utilisation dans l'amplification d'une immunoactivité de mammifères.

2. Immunopotentialisateur pour utilisation selon la revendication 1, dans lequel l'acide nucléique comprend une séquence GATC dans laquelle le A représente une base N⁶-méthyladénine.

3. Immunopotentialisateur pour utilisation selon la revendication 1 ou la revendication 2, dans lequel l'acide nucléique est un acide nucléique comportant une séquence de bases de SEQ ID NO : 4.

4. Immunopotentialisateur pour utilisation selon l'une quelconque des revendications 1 à 3, qui comprend en outre en tant que principe actif un acide nucléique contenant une séquence CpG non méthylée spécifique de l'acide nucléique microbien ou un plasmide comportant l'acide nucléique contenant la séquence CpG non méthylée spécifique de l'acide nucléique microbien.

5. Immunopotentialisateur pour utilisation selon la revendication 4, dans lequel l'acide nucléique contenant la séquence CpG non méthylée spécifique de l'acide nucléique microbien est un acide nucléique comportant la séquence de bases de SEQ ID NO : 2.

6. Immunopotentialisateur pour utilisation selon l'une quelconque des revendications 1 à 5, dans lequel le microbe est un virus ou une bactérie.

7. Immunopotentialisateur pour utilisation selon la revendication 6, dans lequel la bactérie est Escherichia coli.

8. Immunopotentialisateur tel que défini dans l'une quelconque des revendications 1, 2, 3, 6 ou 7, conjointement avec une composition comprenant en tant que principe actif un acide nucléique contenant une séquence CpG non méthylée spécifique de l'acide nucléique microbien ou un plasmide comportant l'acide nucléique contenant la séquence CpG non méthylée spécifique de l'acide nucléique microbien pour utilisation dans l'amplification d'une immunoactivité de mammifères, dans lequel ledit immunopotentialisateur et ladite composition sont administrés simultanément à des mammifères.

9. Utilisation d'un immunopotentialisateur tel que défini dans l'une quelconque des revendications 1 à 7 dans la fabrication d'un médicament pour l'amplification de l'immunoactivité d'un mammifère.

10. Utilisation d'un immunopotentialisateur tel que défini dans l'une quelconque des revendications 1, 2, 3, 6 ou 7, conjointement avec une composition comprenant en tant que principe actif un acide nucléique contenant une séquence CpG non méthylée spécifique de l'acide nucléique microbien ou un plasmide comportant l'acide nucléique contenant la séquence CpG non méthylée spécifique de l'acide nucléique microbien dans la fabrication d'un médicament pour l'amplification de l'immunoactivité d'un mammifère, dans laquelle ledit immunopotentialisateur et ladite composition sont administrés simultanément à des mammifères.

11. Procédé de production d'une cytokine inflammatoire, qui comprend l'administration de l'immunopotentialisateur tel que défini dans l'une quelconque des revendications 1 à 7 à des cellules cultivées pour amplifier une immunoactivité des cellules cultivées et produire la cytokine inflammatoire.

12. Procédé de production d'une cytokine inflammatoire, qui comprend l'administration simultanée aux cellules cultivées de l'immunopotentialisateur tel que défini dans l'une quelconque des revendications 1, 2, 3, 6 ou 7, conjointement avec une composition comprenant en tant que principe actif un acide nucléique contenant une séquence CpG non méthylée spécifique de l'acide nucléique microbien ou un plasmide comportant l'acide nucléique contenant la séquence CpG non méthylée spécifique de l'acide nucléique microbien pour amplifier davantage une immunoactivité et produire la cytokine inflammatoire.

13. Immunopotentialisateur pour mammifères, qui comprend en tant que principe actif un acide nucléique contenant une base modifiée spécifique de l'acide nucléique microbien qui est une base N⁶-méthyladénine, dans lequel ledit acide nucléique comporte une séquence de bases de SEQ ID NO : 4, un dérivé de celui-ci ou un plasmide comportant ledit acide nucléique.

14. Immunopotentialisateur selon la revendication 13, qui comprend en outre les caractères tels que définis dans l'une quelconque des revendications 4 à 7.


### SEQUENCE LISTING

<110> Japan Science and Technology Agency
<120> Immunity activity reinforcement agent
<130> NP03416-JN
<160> 4
<170> PatentIn version 3. 1
<210> 1
   <211> 20
   <212> DNA
   <213> Artificial Sequence
<220>
   <223> GpC-ODN1720, the motif is "non-CpG"
<400> 1
   tccatgagct tcctgatgct 20
<210> 2
   <211> 20
   <212> DNA
   <213> Artificial Sequence
<220>
   <223> CpG-ODN1668, the motif is "CpG"
<400> 2
   tccatgacgt tcctgatgct 20
<210> 3
   <211> 20
   <212> DNA
   <213> Artificial Sequence
<220>
   <223> GATC-dA, the motif is "non-m6A"
<400> 3
   tccatgatct tcctgatgct 20
<210> 4
   <211> 20
   <212> DNA
   <213> Artificial Sequence
<220> n=N6-methyladenin (m6A)
   <223> GATC-m6A, the motif is "m6A"
<400> 4
   tccatgntct tcctgatgct 20